Europäisches Patentamt

European Patent Office

Office européen des brevets

(19)

(11) Veröffentlichungsnummer: **0 240 874**
**B1**

(12) **EUROPÄISCHE PATENTSCHRIFT**

(45) Veröffentlichungstag der Patentschrift: **12.09.90**

(51) Int. Cl.⁵: **A 61 K 31/37**

(21) Anmeldenummer: **87104600.9**

(22) Anmeldetag: **27.03.87**

(54) **Hochresorbierbare Zubereitungsform des Hymecromons und Verfahren zur Herstellung derselben.**

(30) Priorität: **05.04.86 DE 3611467**

(43) Veröffentlichungstag der Anmeldung:
**14.10.87 Patentblatt 87/42**

(45) Bekanntmachung des Hinweises auf die
Patenterteilung:
**12.09.90 Patentblatt 90/37**

(84) Benannte Vertragsstaaten:
**AT BE CH DE ES FR GB GR IT LI LU NL SE**

(56) Entgegenhaltungen:
**DE-A-2 700 085**
**US-A-3 175 943**

(73) Patentinhaber: **Dolorgiet GmbH & Co. KG**
**Otto-von-Guericke-Str. 1**
**5205 St. Augustin 3 (DE)**

(72) Erfinder: **Löhner, Manfred, Chem.**
**Blücherstrasse 47**
**D-5300 Bonn 1 (DE)**

(74) Vertreter: **Werner, Hans-Karsten, Dr. et al**
**Deichmannhaus am Hauptbahnhof**
**D-5000 Köln 1 (DE)**

Courier Press, Leamington Spa, England.

**Beschreibung**

Gegenstand der Erfindung ist eine hochresorbierbare Zubereitungsform des Hymecromons, Verfahren zur Herstellung derselben sowie deren Verwendung als Füllmaterial von Kapseln, vorzugsweise Weichgelatinekapseln.

Die Verbindung Hymecromon, 7-Hydroxy-4-methyl-cumarin, ist z.B. aus Martindale, The Extra Pharmacopoeia, 28th edition, 1982, S. 651 als Arzneistoff bekannt, der gallensekretionsfördernde und gallenweg-krampflösende Eigenschaften besitzt. Er wird eingesetzt zur Behandlung von Funktionsstörungen im Leber-Galle-Bereich, insbesondere bei Krampfzuständen und Dyskinesien der Gallenwege, sowie bei funktionellen Beschwerden nach Eingriffen im Bereich der Gallenwege. Die genannten Beschwerden treten häufig akut auf und sind zudem oft mit heftigen Schmerzen verbunden.

Von einem Arzneimittel, das sich zur Bekämpfung akuter Beschwerden eignet, wird ein rascher Wirkungseintritt gefordert, der wiederum nur durch eine rasche Freisetzung und gute Bioverfügbarkeit des Wirkstoffes erreicht wird. Bislang ist Hymecromon zur enteralen Verabreichung nur in fester Form in Dragées oder Tabletten im Handel.

Hymecromon ist in kaltem und warmem Wasser nahezu unlöslich (vgl. Beilsteins Handbuch der Organischen Chemie, 4. Aufl. 1934, Bd. 18, S. 32) und somit die Resorption im wässrigen Milieu des Magens sehr erschwert. Damit bei festen Darreichungsformen, wie den handelsüblichen, eine gute Verfügbarkeit des Hymecromons gewährleistet ist, müssen hohe Anforderungen an die physikalischen Eigenschaften des Wirkstoffes, wie Teilchengröße und spezifische Oberfläche gestellt werden. Vor allem müssen die Herstellungsbedingungen der Tabletten und Dragéekerne streng eingehalten werden, da bereits geringe Veränderungen des Produktionsablaufes, wie Mischen, Preßdruck oder Maschinentyp, die physikalischen Eigenschaften der Wirkstoffteilchen beeinflussen und die Bioverfügbarkeit beeinträchtigen.

Aus der US—PS—3,175,943 sind Tabletten sowohl mit freiem Hymecromon als auch mit dem Natriumsalz bekannt. Die hier außerdem erwähnte Lösung von Hymecromon in Propylenglykol, zu der Angaben zur Konzentration fehlen, diente offenbar nur zur Prüfung der pharmakologischen Wirkung.

Aus Der DE—OS—2 700 085 ist ein Arzneimittel mit den Wirkstoffen Hymecromon und Chenodesoxychlosäure und die Verwendung dieser Wirkstoffkombination in Kapseln bekannt. Hierbei werden durch die ähnlichen Eigenschaften des Hymecromons (Verminderung der Cholesterinkonzentration der Galle) und die ergänzenden Eigenschaften des Hymecromons (spasmolytische und choleretische Wirkung) die Wirkungen der Chenodesoxycholsäure verbessert.

Der Erfindung liegt zunächst die Aufgabe zugrunde, ein gut einnehmbares Arzneimittel zur Verfügung zu stellen, welches eine wirksame Menge Hymecromon in einem Träger enthält, das einfach herstellbar ist und rasch eine hohe Wirksamkeit entfaltet. Hymecromon ist zwar in einigen physiologisch verträglichen Lösungsmitteln ein wenig, das Natriumsalz jedoch in Wasser gut löslich. Beim Einbringen derartiger Lösungen in wässrige Medien, wie z.B. Magensaft, fällt das Hymecromon jedoch sofort aus, wobei die Bildung grober Kristalle durch Einbau von Kristallwasser begünstigt wird. Gelangt also eine solche Lösung nach oraler Einnahme in den Magen, so bewirkt der wässrige und zudem noch saure Mageninhalt eine Ausfüllung und auschließende Kristallisation des Hymecromons das somit einer schnellen Resorption entzogen wird.

Aus Hagers Handbuch der Pharmazeutischen Praxis, 4. Ausgabe, Springer Verlag 1977, Seiten 403—409, sind Polyethylenglykole und Polyethylen-polypropylenglykole als Trägerstoffe bekannt, von denen einige Typen auch für flüssige Zubereitungsformen geeignet sind. Es wurde jetzt gefunden, daß sich die Löslichkeit von Hymecromon in Verdauungssäften erheblich steigern läßt, wenn der Wirkstoff in einem Gemisch aus Polyalkylenglykol, mindestens einem Tensid und gegebenenfalls 1,2-Propandiol teils gelöst und teils in nichtkristalliner Form suspendiert vorliegt.

Als Polyalkylenglykole kommen insbesondere Polyethylenglykol und/oder Polypropylenglykol in Frage, wobei die Molmassen im Bereich zwischen 200 und 1000, vorzugsweise sogar in dem Bereich zwischen 200 und 630 liegen. Weiterhin kommt Polyoxiethylen-polyoxipropylen-polymer in Frage, dessen Molmassenbereich zwischen 1400 und 2000 liegt. Dieses Polyalkylenglykol hat obendrein die Eigenschaft als Tensid zu wirken, so daß gegebenenfalls sogar auf den Zusatz weiterer Tenside verzichtet werden kann. Weitere besonders geeignete Tenside sind Polyoxiethylen-glycerol-tri-hydroxistearat, Polyoxiethylen-($C_{12-18}$)-fett-alkoholether, Polyoxiethylen-stearat, Polyoxiethylen-sorbitan-mono-($C_{12-18}$)-fett-säureester, Natrium-dioctyl-sulfosuccinat oder Gemische derselben.

Vorzugsweise liegen die Ethylenoxideinheiten des Polyoxiethylenglycerol-tri-hydroxistearats zwischen 35 und 65, die des Polyoxiethylen-($C_{12-18}$)-fettalkoholethers zwischen 15 und 25, die Ethylenoxideinheiten des Polyoxiethylen-stearats zwischen 15 bis 45 und die des Polyoxiethylen-sorbitan-mono-($C_{12-18}$)-fettsäureesters zwischen 15 und 25.

All diese Polyalkylenglykole und Tenside sind im Handel erhältlich. Sie sind physiologisch verträglich und deshalb als Hilfsmittel zur Verwendung pharmazeutischer Zubereitungen geeignet.

Um die Löslichkeit des Hymecromons in den Polyethylenglykolen und Tensiden zu erhöhen, kann es zweckmäßig sein, gegebenenfalls etwas 1,2-Propandiol zuzusetzen.

Das Hymecromon liegt in den erfindungsgemäßen Zubereitungen in Mengen von 20 bis 45 Gewichtsteilen Wirkstoff und 55 bis 80 Gewichtsteilen der Gemische vor. Bei Verwendung von Polyoxiethylen-polyoxipropylen-polymer werden für 20 bis 45 Gewichtsteile Hymecromon, meist 55 bis 80 Gewichtsteile des Polyalkylenglykols und gegebenenfalls bis zu 2 Gewichtsteile 1,2-Propandiol zugegeben. Bei Verwendung von anderen Polyalkylenglykolen werden 20 bis 45 Gewichtsteile Hymecromon in 25 bis 65 Gewichtsteilen des Polyalkylenglykols, vorzugsweise Polyethylenglykol, 5 bis 30 Gewichtsteile Tensid bzw. Tensiden sowie bis zu 2 Gewichtsteilen 1,2-Propandiol gelöst. Dazu wird der Wirkstoff bei Temperaturen von 60 bis 80°C in das Gemisch eingearbeitet und in eine nichtkristalline Form umgewandelt. Ausschlaggebend dabei ist, daß ein Teil des Hymecromons beim Abkühlen auf Raumtemperatur gelöst bleibt und der nicht gelöste Teil nicht in seine ursprüngliche Kristallform zurückkehrt. Es wurde weiterhin gefunden, daß beim Einbringen dieser Suspensionen in wässrige Medien, insbesondere künstlichen Magensaft, keine Rekristallisation erfolgt, so daß das Hymecromon aus dieser Suspension heraus rasch und vollständig resorbiert werden kann. Die Suspensionen können obendrein in an sich bekannter Weise in Kapseln, vorzugsweise Weichgelatinekapseln eingearbeitet werden. Diese Darrichungsform weist gegenüber allen bisher bekannten Darreichungsformen des Hymecromons den Vorteil auf, daß nach der Einnahme der Wirkstoff rasch resorbiert wird.

Dabei ist es nicht notwendig, den Wirkstoff nach der Synthese durch aufwendige galenische Maßnahmen zu verarbeiten. Trotzdem erhält man zuverlässig reproduzierbare hohe Bioverfügbarkeiten und damit einen raschen und zugerlässigen Wirkungseintritt.

Bei der Durchführung des erfindungsgemäßen Verfahrens wird das Hymecromon bei 60 bis 80°C in das Gemisch eingearbeitet, wobei es sich entweder vollständig löst und beim Abkühlen auf Raumtemperatur teilweise wieder ausfällt. Es kann sogar genügen, den Wirkstoff einige Zeit bei 60 bis 80°C in dem Gemisch intensiv zu rühren, um eine nichtkristalline Suspension zu erhalten. Das Lösungsmittel-Tensidgemisch wird dazu zuvor durch homogenes Mischen der Bestandteile bei Temperaturen bis zu 100°C hergestellt.

Gegenstand der Erfindung sind somit zunächst die hochresorbierbaren Zubereitungsformen gemäß den Patentansprüchen 1 bis 5 sowie ein Verfahren zur Herstellung derselben gemäß, den Ansprüchen 6 bis 10. Schließlich betrifft die Erfindung noch die Verwendung der Zubereitungen gemäß den Ansprüchen 1 bis 5 als Füllmaterial von Kapseln, vorzugsweise Weichgelatinekapseln.

In den folgenden Beispielen sind die erfindungsgemäßen Zubereitungen sowie Verfahren zu ihrer Herstellung näher erläutert.

### Beispiel 1

Man löst unter Rühren bei 70 bis 75°C 1,24 kg Polyoxiethylen-(40)-glycerol-tri-hydroxistearat und 0,29 kg 1,2-Propandiol in 11,47 kg Polyoxiethylen-polyoxipropylen-Polymer 1900 und fügt dieser Lösung portionsweise unter Rühren 8,00 kg Hymecromon zu. Anschließend wird die Mischung mittels eines Hochleistungs-Dispergiergerätes homogenisiert und allmählich auf Raumtemperatur abgekühlt.

1,050 g Suspension enthalten 400 mg Hymecromon.

### Beispiel 2

Man löst unter Rühren bei 95 bis 100°C nacheinander 1,24 kg Polyoxiethylen-(40)-glycerol-tri-hydroxistearat, 0,29 kg 1,2-Propandiol und 1,00 kg Natrium-dioctyl-sulfosuccinat in 11,47 kg Polyoxiethylen-polyoxipropylen-Polymer 1900, kühlt auf 70 bis 75°C ab und fügt dieser Lösung portionsweise unter Rühren 8,00 kg Hymecromon zu. Anschließend wird die Mischung mittels eines Hochleistungs-Dispergiergerätes homogenisiert und allmählich auf Raumtemperatur abgekühlt.

1,100 g Suspension enthalten 400 mg Hymecromon.

### Beispiel 3

Analog Beispiel 2 wird eine Suspension hergestellt aus 57,35 g Polyoxiethylen-polyoxipropylen-Polymer 1900, 1,45 g 1,2-Propandiol, 6,20 g Polyoxiethylen-(40)-glycerol-tri-hydroxistearat, 5,00 g Natrium-dioctyl-sulfosuccinat und 20,00 g Hymecromon.

0,900 g Suspension enthalten 200 mg Hymecromon.

### Beispiel 4

Analog Beispiel 1 wird eine Suspension hergestellt aus 57,35 g Polyethylenglykol 200, 1,45 g 1,2-Propandiol, 6,20 g Polyoxiethylen-(40)-glycerol-tri-hydroxistearat und 40,00 g Hymecromon.

1,050 g Suspension enthalten 400 mg Hymecromon.

### Beispiel 5

Analog Beispiel 1 wird eine Suspension hergestellt aus 28,675 g Polyethylenglykol 400, 28,67 g Polyoxiethylen-polyoxipropylen-Polymer 1900, 1,45 g 1,2-Propandiol und 40,00 g Hymecromon.

0,988 g Suspension enthalten 400 mg Hymecromon.

### Beispiel 6

Analog Beispiel 1 wird eine Suspension hergestellt aus 57,35 g Polyoxiethylen-polyoxipropylen-Polymer 1900, 1,45 g 1,2-Propandiol und 40,00 g Hymecromon.

0,988 g Suspension enthalten 400 mg Hymecromon.

Beispiel 7

Analog Beispiel 1 wird eine Suspension hergestellt aus 57,35 g Polyoxiethylen-polyoxipropylen-Polymer 1900, 1,45 g 1,2-Propandiol und 20,00 g Hymecromon.

0,788 g Suspension enthalten 200 mg Hymecromon.

Beispiel 8

Analog Beispiel 1 wird eine Suspension hergestellt aus 57,35 g Polyoxiethylen-polyoxipropylen-Polymer 1900, 1,45 g 1,2-Propandiol, 6,20 g Polyoxiethylen-(30)-stearat und 40,00 g Hymecromon.

1,050 g Suspension enthalten 400 mg Hymecromon.

Beispiel 9

Analog Beispiel 1 wird eine Suspension hergestellt aus 57,35 g Polyoxiethylen-polyoxipropylen-Polymer 1900, 1,45 g 1,2-Propandiol, 6,20 g Polyoxiethylen-(20)-stearylalkohol und 40,00 g Hymecromon.

1,050 g Suspension enthalten 400 mg Hymecromon.

Beispiel 10

Analog Beispiel 1 wird eine Suspension hergestellt aus 57,35 g Polyoxiethylen-polyoxipropylen-Polymer 1500, 1,45 g 1,2-Propandiol, 6,20 g Polyoxiethylen-(20)-sorbitan-monostearat und 40,00 g Hymecromon.

1,050 g Suspension enthalten 400 mg Hymecromon.

Beispiel 11

Analog Beispiel 1 wird eine Suspension hergestellt aus 57,35 g Polyoxiethylen-polyoxipropylen-Polymer 2000, 1,45 g 1,2-Propandiol, 6,20 g Polyoxiethylen-(60)-glycerol-tri-hydroxistearat und 40,00 g Hymecromon.

1,050 g Suspension enthalten 400 mg Hymecromon.

Beispiel 12

Analog Beispiel 2 wird eine Suspension hergestellt aus 57,35 g Polyoxiethylen-polyoxipropylen-Polymer 1900, 1,45 g 1,2-Propandiol, 6,20 g Natrium-dioctyl-sulfosuccinat und 40,00 g Hymecromon.

1,050 g Suspension enthalten 400 mg Hymecromon.

Beispiel 13

Analog Beispiel 2 wird eine Suspension hergestellt aus 57,35 g Polyethylenglykol 400, 1,45 g 1,2-Propandiol, 6,20 g Polyoxiethylen-(40)-glycerol-tri-hydroxistearat, 5,00 g Natrium-dioctyl-sulfosuccinat und 40,00 g Hymecromon.

1,100 g Suspension enthalten 400 mg Hymecromon.

Die Freisetzung des Hymecromons aus den erfindungsgemäßen Präparaten wurde mittels Dissolution-Test nach USP XX, 5th Supplement (Paddle-Methode), in 900 ml künstlichen Magensaft entsprechend USP XX ohne Pepsin bei 36,5°C geprüft. Die Rotationsgeschwindigkeit des Rührers betrug 100 UpM. Das Freigabemedium wurde kontinuierlich über eine mit einem Filter versehene Glasfritte (G3) mittels einer Schlauchpumpe durch die Durchflußküvette eines Beckman-Spektralphotometers, Modell DU-7, und züruck ins Freigabegefäß gepumpt. Die Konzentration des Hymecromons wurde bei der Wellenlänge von 257 nm bestimmt. Die in der Tabelle angegebenen Mittelwerte wurden aus je 6 Einzelmessungen errechnet.

Zum Vergleich werden ein handelsübliches Dragée mit 400 mg Hymecromon und eine Tablette gemäß der US—PS—3,175,943 mit 450 mg Hymecromon-Natriumsalz (entsprechend 400 mg Hymecromon) herangezogen.

Aus der Tabelle 1 ist zu ersehen, daß das Hymecromon aus den erfindungsgemäßen Präparaten wesentlich schneller freigesetzt wird als aus dem handelsüblichen Dragée und aus der US—PS—3,175,943 bekannten Tablette. Außerdem kommt die Freisetzung des Hymecromons aus den Vergleichspräparaten nach 40 Minuten bei ca. 35 bis 41% zum Stillstand während sie bei den erfindungsgemäßen Präparaten Werte bis zu 67% erreicht.

4

## Tabelle 1

| Präparat | Freisetzung von Hymecromon in % nach .. Minuten | | | | | | | | | |
|---|---|---|---|---|---|---|---|---|---|---|
| | 6 | 7 | 8 | 10 | 12 | 14 | 16 | 20 | 30 | 40 |
| Handelsübliches Dragée | 0,5 | 0,6 | 0,7 | 1,8 | 3,4 | 9,1 | 14,8 | 23,9 | 32,9 | 35,8 |
| Tablette nach US-PS 3,175,943 | 2,5 | 9,7 | 17,2 | 28,3 | 32,4 | 34,0 | 37,9 | 37,9 | 39,6 | 41,1 |
| Beispiel 1 | 3,7 | 23,1 | 32,3 | 42,8 | 47,5 | 50,1 | 51,7 | 53,2 | 54,4 | 54,7 |
| Beispiel 2 | 7,8 | 23,6 | 34,5 | 48,9 | 52,6 | 54,1 | 54,7 | 54,6 | 55,2 | 55,4 |
| Beispiel 4 | 6,2 | 27,7 | 40,4 | 49,2 | 51,6 | 52,6 | 52,9 | 53,5 | 53,8 | 53,9 |
| Beispiel 5 | 0,9 | 9,1 | 20,6 | 33,9 | 41,6 | 45,6 | .47,6 | 49,6 | 51,3 | 51,9 |
| Beispiel 6 | 3,4 | 23,2 | 38,6 | 46,7 | 49,6 | 50,9 | 51,7 | 52,5 | 53,2 | 53,5 |
| Beispiel 8 | 0,3 | 2,9 | 7,6 | 19,2 | 32,4 | 41,7 | 47,3 | 51,8 | 53,9 | 54,3 |
| Beispiel 9 | 3,6 | 13,9 | 28,5 | 47,4 | 52,2 | 53,5 | 54,1 | 54,6 | 54,9 | 54,9 |
| Beispiel 10 | 1,3 | 7,2 | 13,7 | 29,7 | 41,5 | 46,6 | 49,5 | 52,2 | 53,8 | 54,2 |
| Beispiel 11 | 2,2 | 7,6 | 13,3 | 31,5 | 41,8 | 47,2 | 50,0 | 52,4 | 53,9 | 54,3 |
| Beispiel 12 | 3,4 | 13,4 | 29,3 | 48,3 | 53,9 | 58,1 | 61,7 | 64,3 | 66,2 | 67,3 |
| Beispiel 13 | 3,8 | 21,6 | 37,6 | 46,8 | 49,6 | 50,9 | 51,6 | 52,3 | 53,1 | 53,4 |

EP 0 240 874 B1

Die Suspensionen gemäß den Ansprüchen 1 bis 5 ließen sich ohne Schwierigkeiten in an sich bekannter Weise in Weichgelatinekapseln einbringen und behielten auch in dieser Form ihre gute Resorbierbarkeit. Selbstverständlich können sie auch in Hartgelatinekapseln oder andere Kapseln eingebracht werden, da auch diese sich inzwischen mit Suspensionen füllen lassen.

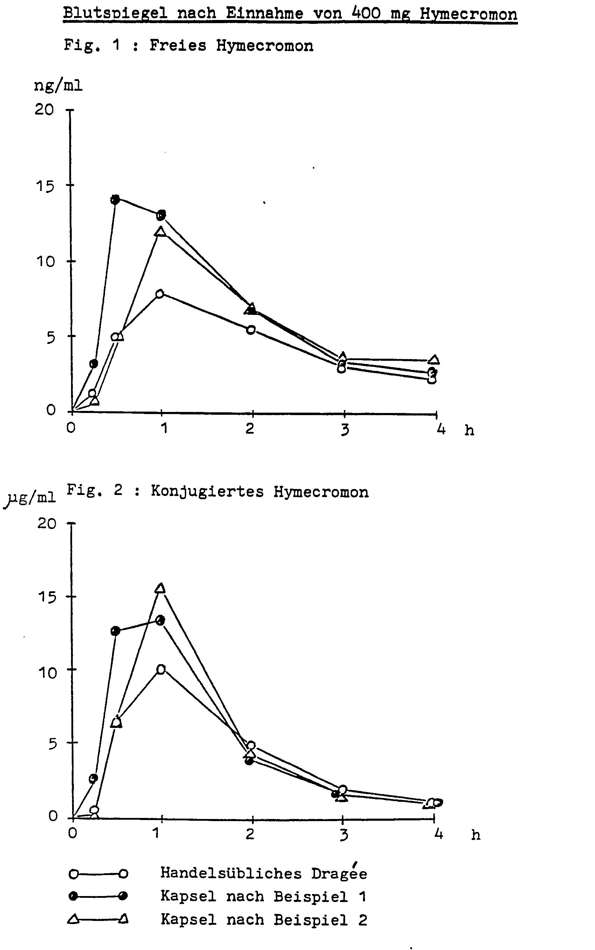

Die Bioverfügbarkeit des Hymecromons aus den erfindungsgemäßen Kapseln und einem handelsüblichen Dragée wurde an fünf Probanden geprüft. Dazu wurde den Versuchspersonen einmalig eine Kapsel bzw. ein Dragée mit jeweils 400 mg Hymecromon verabreicht. Vor der Einnahme sowie nach 0,25, 0,5, 1, 2, 3 und 4 Stunden wurde den Probanden Blut abgenommen. Aus dem Blut wurde durch Zentrifugieren unter Zusatz von Ethylendiaminotetraessigsäure Plasma gewonnen. Im menschlichen Organismus wird Hymecromon rasch mit Glukuron- bzw. Schwefelsäure konjugiert, so daß im Plasma neben freiem (nichtkonjugiertem) Hymecromon die Konjugate erscheinen. Bei der obigen Prüfung wurde sowohl freies als auch konjugiertes Hymecromon, letzteres nach Spaltung mit β-Glukuronidase/Arylsulfatase, über HPLC-Trennung mit einem elektrochemischen Detektor bestimmt. Die Mittelwerte der Bestimmungen sind in den Figuren 1 und 2 als Kurven dargestellt.

Unter der Bioverfügbarkeit eines Arzneistoffes aus einer Zubereitung versteht man die Geschwindigkeit und das Ausmaß, mit denen dieser in die Blutbahn gelangt (E. Mutschler, Arzneimittel-Wirkungen, Wissenschaftliche Verlagsgesellschaft mbH, Stuttgart 1986, Seite 46). Nach F. H. Dost (Grundlagen der Pharmakokinetik, Georg Thieme Verlag, Stuttgart 1968, Seite 155) entspricht die Fläche, welche die Blutspiegelkurve mit der Zeitachse umschließt (AUC = area under the curve) der Substanzmenge im Organismus. Daraus ergibt sich die Möglichkeit, die Bioverfügbarkeit einer Substanz aus verschiedenen Darreichungsformen zu vergleichen. Aus den in den Figuren 1 und 2 dargestellten Plasmaspiegelverläufen wurden die AUC-Werte berechnet und die relative Bioverfügbarkeit des Hymecromons ermittelt (Tabelle 2).

Tabelle 2 zeigt, daß mit den erfindungsgemäßen Kapseln die Bioverfügbarkeit des Hymecromons gegenüber dem handelsüblichen Dragée um bis zu 51% verbessert wurde. Darüber hinaus ist, wie aus dem Vergleich des Anstiegs der Blutspiegelkurven der Figuren 1 und 2 ersichtlich ist, bei den erfindungsgemäßen Kapseln eine schnellere Anflutung des Wirkstoffes als bei dem vorbekannten Präparat gegeben. Mit der schnelleren und höheren Freisetzung und Bioverfügbarkeit von Hymecromon aus den erfindungsgemäßen Kapseln steht somit ein besser und schneller wirksames, Hymecromon enthaltendes Arzneimittel als bisher zur Verfügung.

## Tabelle 2

| | Freies Hymecromon | | Konjugiertes Hymecromon | |
|---|---|---|---|---|
| | AUC-Werte h x ng/ml | Relative Bioverfüg-barkeit | AUC-Werte h x µg/ml | Relative Bioverfüg-barkeit |
| Kapsel entspr. Beispiel 1 | 27,74 | 1,51 | 22,03 | 1,23 |
| Kapsel entspr. Beispiel 2 | 23,76 | 1,29 | 21,02 | 1,17 |
| Handelsübliches Dragée (400 mg) | 18,38 | 1 | 17,90 | 1 |

**Patentansprüche**

1. Hochresorbierbare Zubereitungsform des Hymecromons, dadurch gekennzeichnet, daß 20 bis 45 Gewichtsteile des Wirkstoffs in 55 bis 80 Gewichtsteilen eines Gemisches aus Polyalkylenglykol, mindestens einem Tensid und gegebenenfalls bis zu 2 Gewichtsteilen 1,2-Propandiol teils gelöst und teils in nichtkristalliner Form suspendiert vorliegen.

2. Zubereitungsform gemäß Anspruch 1, dadurch gekennzeichnet, daß als Polyalkylenglykol und als Tensid Polyoxiethylen-polyoxipropylen-polymer mit Molmassen von 1400 bis 2000 verwendet wird.

3. Zubereitungsform gemäß Anspruch 1, dadurch gekennzeichnet, daß als Polyalkylenglykol Polyethylenglykol und/oder Polypropylenglykol mit Molmassen von 200 bis 100 verwendet werden.

4. Zubereitungsform gemäß einem der Anspruche 1 bis 3, dadurch gekennzeichnet, daß als Tenside Polyoxiethylen-glycerol-tri-hydroxistearat, Polyoxiethylen-($C_{12-18}$)-fett-alkoholether, Polyoxiethylen-stearat, Polyoxiethylen-sorbitan-mono-($C_{12-18}$)-fettsäureester, Natrium-dioctyl-sulfosuccinat oder Gemische derselben verwendet werden.

5. Zubereitungsform gemäß Anspruch 4, dadurch gekennzeichnet, daß die Ethylenoxideinheiten des Polyoxiethylen-glycerol-tri-hydroxistearats zwischen 35 und 65, die des Polyoxiethylen-(C$_{12-18}$)-fettalkoholethers zwischen 15 und 25, die Ethylenoxideinheiten des Polyoxiethylen-stearats zwischen 15 bis 45 und die des Polyoxiethylen-sorbitan-mono-(C$_{12-18}$)-fettsäureesters zwischen 15 und 25 liegen.

6. Verfahren zur Herstellung hochresorbierbarer Zubereitungsformen des Hymecromons, dadurch gekennzeichnet, daß 20 bis 45 Gewichtsteile des Wirkstoffes bei Temperaturen zwischen 60 und 80°C in einem Gemisch aus 55 bis 80 Gewichtsteilen Polyalkylenglykol, mindestens einem Tensid und gegebenenfalls bis zu 2 Gewichtsteilen 1,2-Propandiol gelöst werden und die Lösung auf Raumtemperatur abgekühlt wird.

7. Verfahren gemäß Anspruch 6, dadurch gekennzeichnet, daß als Polyalkylenglykol und als Tensid Polyoxiethylen-polyoxipropylen-polymer mit Molmassen von 1400 bis 2000 verwendet wird.

8. Verfahren gemäß Anspruch 6, dadurch gekennzeichnet, daß als Polyalkylenglykol Polyethylenglykol und/oder Polypropylenglykol mit Molmassen von 200 bis 1000 verwendet werden.

9. Verfahren gemäß einem der Ansprüche 6 bis 8, dadurch gekennzeichnet, daß als Tenside Polyoxiethylen-glycerol-tri-hydroxistearat, Polyoxiethylen-(C$_{12-18}$)-fettalkoholether, Polyoxiethylen-stearat, Polyoxiethylensorbitan-mono-(C$_{12-18}$)-fettsäureester, Natrium-dioctyl-sulfosuccinat oder Gemische derselben verwendet werden.

10. Verfahren gemäß Anspruch 9, dadurch gekennzeichnet, daß die Ethylenoxideinheiten des Polyoxiethylenglycerol-tri-hydroxistearats zwischen 35 und 65, die des Polyoxiethylen-(C$_{12-18}$)-fettalkoholethers zwischen 15 und 25, die Ethylenoxideinheiten des Polyoxiethylenstearats zwischen 15 bis 45 und die des Polyoxiethylen-sorbitan-mono-(C$_{12-18}$)-fettsäureesters zwischen 15 und 25 liegen.

11. Verwendung der Zubereitung formen gemäß den Ansprüchen 1 bis 5 als Fullmaterial von Kapseln.

**Revendications**

1. Préparation d'hymecromone à résorption élevée, caractérisée en ce que 20 à 45 parties en poids de matière active sont dissoutes dans 55 à 80 parties en poids d'un mélange de polyakylène-glycol, d'au moins un produit tensioactif et, le cas échéant, de jusqu'à 2 parties en poids de propanediol-1,2, et se trouvent en partie en suspension sous une forme non cristalline.

2. Préparation selon la revendication 1, caractérisée en ce qu'on utilise comme polyalylène-glycol et comme tensioactif, un polymère polyoxyéthylène-polyoxypropylène avec des masses molaires allant de 1400 à 2000.

3. Préparation selon la revendication 1, caractérisée en ce qu'on utilise comme polyalkylène-glycol du polyéthylène-glycol et/ou du polypropylène-glycol avec des masses molaires de 200 à 1000.

4. Préparation selon l'une des revendications 1 à 3, caractérisé en ce qu'on utilise comme tensioactifs, du trihydroxystéarate de polyoxyéthylène-glycérol, un éther de polyoxyéthylène-acide gras (C$_{12}$—C$_{18}$), du stéarate de polyoxyéthylène, un ester de polyoxyéthylène-sorbitan-mono acide gras (C$_{12}$—C$_{18}$), du dioctyl-sulfosuccinate de sodium ou des mélanges de ceux-ci.

5. Préparation selon la revendication 4, caractérisée en ce que les unités oxyde d'éthylène du trihydroxystéarate de polyoxyéthylène-glycérol sont comprises entre 35 et 65, celles de l'éther de polyoxyéthylènealcool gras (C$_{12}$—C$_{18}$) entre 15 et 25, les unités oxyde d'éthylène du stéarate de polyoxyéthylène entre 15 et 45 et celles de l'ester de polyoxyéthylène-sorbitan-mono acide gras (C$_{12}$—C$_{18}$) entre 15 et 25.

6. Procédé de préparation d'hymécromone à résorption élevée, caractérisé en ce que 20 à 45 parties en poids de la matière active sont dissoutes à des températures comprises entre 60 et 80° dans un mélange de 55 à 80 parties en poids de polyalkylène-glycol, d'au moins un tensioactif et, le cas échéant, de jusqu'à 2 parties en poids de propanediol-1,2 et en ce que la solution est refroidie à la température ambiante.

7. Procédé selon la revendication 6, caractérisé en ce qu'on utilise, comme polyalkylène-glycol et comme tensioactif, un polymère polyoxyéthylène-polyoxypropylène avec des masses molaires de 1400 à 2000.

8. Procédé selon la revendication 6, caractérisé en ce qu'on utilise, comme polyalkylène-glycol, du polyéthylène-glycol et/ou du polypropylène-glycol avec des masses molaires de 200 à 1000.

9. Procédé selon l'une des revendications 6 à 8, caractérisé en ce qu'on utilise, comme tensioactif, du tri-hydroxystéarate de polyoxyéthylène-glycérol, un éther de polyoxyéthylène-alcool gras (C$_{12}$—C$_{18}$), du stéarate de polyoxyéthylène, un ester de polyoxyéthylène-sorbitan-mono acide gras (C$_{12}$—C$_{18}$), du dioctyl-sulfosuccinate de sodium ou des mélanges de ceux-ci.

10. Procédé selon la revendication 9, caractérisé en ce que les unités oxyde d'éthylène du trihydroxystéarate de polyoxyéthylène-glycérol sont comprises entre 35 et 65, celles de l'éther de polyoxyéthylène-alcool gras (C$_{12}$—C$_{18}$) entre 15 et 25, les unités oxyde d'éthylène du stéarate de polyoxyéthylène entre 15 et 45 et celles de l'ester du polyoxyéthylène-sorbitan-mono acide gras (C$_{12}$—C$_{18}$) entre 15 et 25.

11. Utilisation de la préparation selon les revendications 1 à 5, comme matière de remplissage de capsules.

**Claims**

1. Highly resorbable preparation form of hymecromone, characterized in that from 20 to 45 parts by weight of the active substance are present, upon having been in part dissolved and in part in a non-crystalline state suspended, in from 55 to 80 parts by weight of a mixture comprising polyalkyleneglycol, at least one surfactant and optionally up to 2 parts by weight of 1,2-propane-diol.

2. The preparation form according to claim 1, characterized in that a polyoxyethylene-polyoxypropylene polymer having a molecular mass of from 1,400 to 2,000 is employed as the polyalkyleneglycol and as the surfactant.

3. The preparaton form according to claim 1, characterized in that polyethyleneglycol and/or polypropyleneglycol having molecular masses of from 200 to 1,000 are employed as the polyalkyleneglycol.

4. The preparation form according to any of claims 1 to 3, characterized in that polyoxyethylene-glycerol-trihydroxystearate, polyoxyethylene-((C$_{12-18}$)-fatty alcohol ethers, polyoxyethylene stearate, polyoxyethylene-sorbitan-mono-(C$_{12-18}$)-fatty acid esters, sodium dioctylsulfosuccinate or mixtures thereof are employed as the surfactant(s).

5. The preparation form according to claim 4, characterized in that the ethylene oxide units of the polyoxyethylene-glycerol-trihydroxystearate are between 35 and 65, the ethylene oxide units of the polyoxyethylene-(C$_{12-18}$)-fatty alcohol ethers are between 15 and 25, the ethylene oxide units of the polyoxyethylene stearate are between 15 and 45, and those of the polyoxyethylene-sorbitan-mono-(C$_{12-18}$)-fatty acid ester are between 15 and 25.

6. A method for the production of highly resorbable preparation forms of hymecromone, characterized in that from 20 to 45 parts by weight of the active substance are dissolved at temperatures between 60°C and 80°C in from 55 to 80 parts by weight of a mixture comprising polyalkyleneglycol, at least one surfactant and optionally up to 2 parts by weight of 1,2-propanediol.

7. The method according to claim 6, characterized in that a polyoxyethylene-polyoxypropylene polymer having a molecular mass of from 1,400 to 2,000 is employed as the polyalkyleneglycol and as the surfactant.

8. The method according to claim 6, characterized in that polyethyleneglycol and/or polypropyleneglycol having molecular masses of from 200 to 1,000 are employed as the polyakyleneglycol.

9. The method according to any of claims 6 to 8, characterized in that polyoxyethylene-glycerol-tri-hydroxystearate, polyoxyethylene-(C$_{12-18}$)-fatty alcohol ethers, polyoxyethylene stearate, polyoxy-ethylene-sorbitan-mono-(C$_{12-18}$)-fatty acid esters, sodium dioctylsulfosuccinate or mixtures thereof are employed as the surfactant(s).

10. The method according to claim 9, characterized in that the ethylene oxide units of the polyoxyethylene-glycerol-trihydroxystearate are between 35 and 65, the ethylene oxide units of the polyoxy-ethylene-(C$_{12-18}$)-fatty alcohol ethers are between 15 and 25, the ethylene oxide units of the polyoxyethylene stearate are between 15 and 45, and those of the polyoxyethylene-sorbitan-mono-(C$_{12-18}$)-fatty acid ester are between 15 and 25.

11. Use of the preparation forms according to the claims 1 to 5 as fill material for capsules.

Blutspiegel nach Einnahme von 400 mg Hymecromon

Fig. 1 : Freies Hymecromon

Fig. 2 : Konjugiertes Hymecromon

○———○   Handelsübliches Dragée
●———●   Kapsel nach Beispiel 1
△———△   Kapsel nach Beispiel 2

1